(19) Europäisches Patentamt / European Patent Office / Office européen des brevets

(11) **EP 2 033 167 B1**

(12) **EUROPEAN PATENT SPECIFICATION**

(45) Date of publication and mention of the grant of the patent:
**04.09.2013 Bulletin 2013/36**

(51) Int Cl.:
*G07C 5/08* (2006.01)  *B60T 1/10* (2006.01)
*B60T 7/04* (2006.01)  *B60T 7/08* (2006.01)
*B60T 7/22* (2006.01)  *B60W 40/072* (2012.01)
*B60W 40/076* (2012.01)  *B60W 40/09* (2012.01)

(21) Application number: **07748514.2**

(22) Date of filing: **24.05.2007**

(86) International application number:
**PCT/SE2007/050353**

(87) International publication number:
**WO 2007/139493 (06.12.2007 Gazette 2007/49)**

(54) **DEVICE FOR DETERMINING AN ANTICIPATION ABILITY OF A DRIVER**

EINRICHTUNG ZUR BESTIMMUNG EINES ANTIZIPATIONSVERHALTENS EINES FAHRERS

DISPOSITIF POUR DÉTERMINER LA CAPACITÉ D'ANTICIPATION D'UN CONDUCTEUR

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.05.2006 SE 0601175**

(43) Date of publication of application:
**11.03.2009 Bulletin 2009/11**

(73) Proprietor: **Scania CV AB (publ)**
**151 87 Södertälje (SE)**

(72) Inventors:
• **BRÅKENHIELM, Erik**
**112 52 Stockholm (SE)**
• **ANDERSSON, Jon**
**151 46 Södertälje (SE)**

(56) References cited:
WO-A-00/07150          WO-A1-00/07150
WO-A1-2006/008731    WO-A2-02/33529
DE-A1- 10 056 756      US-A- 6 092 021

## Description

## Field of the Invention

The present invention relates to systems for determining the anticipation ability of a vehicle driver, and in particular to a device to assess a anticipation ability of the driver of a vehicle according to the preamble of claim 1.

## Background of the Invention

When driving heavy vehicles, such as trucks, buses and the like, the vehicle economy has in time gained a greater effect on the profitability in the business in which the vehicle is used. Except for the acquiring cost of the vehicle, the largest items of expenditure for a vehicle consist of fuel costs and service costs. These costs are often connected, i.e. a vehicle that is heavily used both consumes more fuel and are exposed to greater wear with increasing service costs as a consequence. An existing problem for the companies that uses heavy vehicles in their business is nonetheless that it is difficult to establish how great part of the fuel consumption and wear that for example is derived from incautious driving, and how great amount that is derived from unfavorable traffic surroundings, such as extremely undulated ground and/or urban environment.

The wear a vehicle is exposed to during incautious driving can at least partially be due to the vehicle being exposed to an excessive amount of and unnecessary accelerations and retardations.

In the Patent Application WO00/07150 there is disclosed a system and a method for showing a driver how to drive a vehicle in an efficient manner. For example, the braking force or the acceleration and retardation can be measured to assess whether the driver is driving the vehicle in a favorable manner. The system is using a fixed smallest time interval in which the motor is supposed to be running idle between acceleration and retardation. However, from an environmental point of view, this procedure is not desirable because it is common knowledge that idle running, especially in an environmental and fuel perspective, should be avoided. However, a driver should, of course, motor brake when the accelerator pedal is released before the brake is pressed down. A problem with this system is that it is acceleration and retardation, respectively, that is measured. If the vehicle happens to be in hilly country, the vehicle can first be exposed to retardation and thereafter acceleration (at the top of a hill) without this in any way implying that the driver has had a poor ability to anticipate the traffic.

In the Patent Application DE10056756 there is disclosed a system whereby sensor data related to driving style are collected according to various time windows. Furthermore, there is disclosed means for receiving a parameter value that is representative for the particular vehicle. However, a problem with this system is to have an accurate feedback of the surroundings such as the difference in level of the landscape that the vehicle is driving in.

Accordingly, there exists a need for an improved method to be able to assess the anticipation ability of a vehicle driver.

## Summary of the Invention

It is an object of the present invention to provide a device for determining the anticipation ability of the driver of a vehicle in a traffic situation, which solves the above problem. This and other objects are achieved according to the present invention by a device according to claim 1.

According to the present invention, it is provided a device which comprises means for determining whether the vehicle driver is taking a first measure, whereby said first measure is a braking measure, or a measure for take off of a positive engine torque, for example by requesting an acceleration, means for determining a first time at which said measure stops, means for determining a second time at which the driver is taking a second measure, counteracting said first measure. The device further comprises means for receiving at least one reference parameter value representing the vehicle surroundings, or at least one parameter value from at least one vehicle internal sensor by means of which a reference parameter value, representative for the vehicle surroundings, can be determined, and means for comparing a value representing the difference between said second time and said first time with the reference parameter value, and, based on the comparison, determine a parameter value representing the driver's anticipation ability. Said first and second measure, respectively, can be performed by means of, for example, one from the group: accelerator pedal, accelerator control, brake pedal, brake control, cruise control, retarder, constant-speed brake.

This has the advantage that each time the vehicle driver changes from taking a first type of vehicle actuating measure to a second type of vehicle actuating measure, the time that has lapsed, or the distance the vehicle has traveled in the meantime, can be compared with a corresponding reference time or reference distance that is determined based on the surroundings of the vehicle, i.e. it can be taken into consideration whether the conditions of the surrounding at the change of measure were such that a short intermediate period was desired or even necessary, or if the intermediate period should have been considerably longer. For example, in an urban environment it can be difficult with a longer period between the measures, while a driver on a motorway does not have to be particularly foresighted to be able to fulfill the same longer period. As is realized, a reference distance can simply be transformed into time when the speed of the vehicle is known during the reference distance.

Accordingly, each change of measure that has been performed can be studied in detail to assess whether it was performed in a correct manner, with consideration taken to the traffic environment surroundings.

The device can comprise means for transmitting said parameter value representing the driver's anticipation ability, to display means arranged in said vehicle. This has the advantage that the driver can immediately receive feedback on his/her behavior.

The device can comprise means for storing said determined anticipation ability for a plurality of traffic situations. This has the advantage that, e.g., the driver's average ability during a certain distance, or time period, can be determined. Said parameter values representing the driver's anticipation ability can be transmitted to a distantly located monitoring central. This has the advantage that a vehicle fleet owner centrally can evaluate a plurality of vehicle drivers.

Said traffic situations can consist of any from the group: right-hand turn, left-hand turn, right-hand curve, left-hand curve, straight stretch, roundabout, top of a hill, valley. This has the advantage that a very accurate description of the vehicle surroundings can be used in the said determination, and a very accurate determination can thereby be performed.

The present invention also relates to a vehicle.

**Brief description of the drawings**

Fig. 1 schematically illustrates a control system for a vehicle, where the present invention advantageously can be used.

Fig. 2a-b illustrates an exemplary device according to the present invention.

Fig. 3 illustrates an exemplary method according to the present invention.

Fig. 4 illustrates an alternative exemplary method according to the present invention.

**Detailed description of exemplary embodiments**

In this description, the term vehicle internal sensors means sensors that only obtains data from the relative movements of the vehicle, i.e. changes in movements from one time to another, and which do not use information from external systems, such as positioning systems or map databases. Such vehicle internal sensors can, for example, consist of lateral acceleration sensors, i.e. sensors delivering signals with which the rate of change of the vehicle speed in the lateral direction can be determined, for example in order to determine whether the vehicle runs the risk of tipping over due to the driver performing an incautious maneuver. Furthermore, the sensors can comprise wheel speed sensors, i.e. sensors for delivering signals with which the rotation speed of the wheels and traveled distance can be determined, steering angle sensors that deliver signals with which the angle of the steering wheels in relation to the longitudinal axis of the vehicle can be determined, and sensor means for delivering signals from which the yaw rate, i.e. how quickly the vehicle turns, can be calculated.

With the concept motor torque take off is intended, in this description, a vehicle engine propelling force acting on the vehicle's propulsion. A positive motor torque take off has the meaning that the motor, by means of fuel supply and with a gear in place, generates a force which strives to propel the vehicle forward in the vehicle's direction of motion. Conversely, a negative engine torque take off means motor-braking with a gear in place, which strives to decrease the speed of the vehicle in the direction of motion.

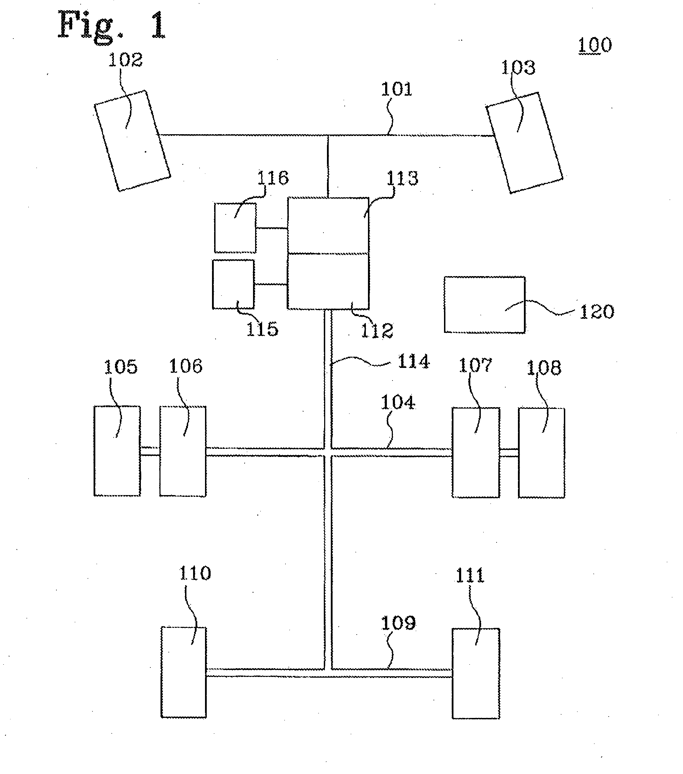

In fig. 1 is schematically illustrated a control system for a vehicle 100 with which the present invention can be utilized. The vehicle 100 comprises a front shaft 101 with steering wheels 102, 103, a rear drive shaft 104 with driving wheels 105-108, and a rear pressure equalizer shaft 109 with wheels 110, 111. Furthermore, the vehicle 100 contains an engine 113 connected to a gear box 112, which drives the drive shaft 104 by means of an output shaft 114 from the gear box.

Gear box 112 and motor 113 are controlled by control units 115, 116, respectively, which are controlled by a main control unit 117. The Engine Management System (EMS) 116 controls the motor functions of the vehicle, which, for example, can consist of fuel injection and engine-brake. The control is based on a number of input signals, which can consist of signals from (not shown) throttle controls (the position of the accelerator pedal), speed sensor and brake management system. The Gearbox Management System (GMS) 115 controls the gear functions, wherein, when using an automatic gearbox, the gear shifting can be controlled based on an input signal from speed sensors, in manual gear shifting the shifting can be controlled from an input signal from a gear selector (gear shift lever).

Furthermore, the vehicle contains a Brake Management System (BMS) with a break control unit 120, which controls the brake functions of the vehicle, such as automatic calculation of the load so that a given pedal position always can result in the same brake effect regardless of the load. The brake control unit controls the various brake systems of the vehicle, e.g. retarder and other supplementary brake systems, exhaust brake and service brake, and sends control signals to system modules (not shown) dispersed on the chassis, where electrical control signals are used, e.g. to adjust brake pressure.

A vehicle of the kind shown in fig. 1 includes, except for the above, typically a number of additional control units, see, for example, WO01/86459 A1. Therefore, the above described control units constitute mere examples of what can exist in a vehicle. As is appreciated by a person skilled in the art, two or more of the above described control units can, of course, be integrated in one single control unit.

In a vehicle of the kind shown in fig. 1, it is, as was mentioned above, highly desirable that the driver, when driving the vehicle, is as foresighted as possible to avoid unnecessary braking actions and accelerations, for example those that have been caused by keeping too short distance to the vehicle ahead, or that the driver requests an acceleration on the top of a hill to then immediately brake in a subsequent downhill slope. It is considerably better, in a wear point of view, as well as from a fuel consumption point of view, if the driver as far as possible takes advantage of energy stored in the vehicle, and do not consume it by using the brakes unnecessarily. The ideal vehicle management by the driver at different situations, however, varies to a large extent, and it is today very difficult to find out whether the driver's behavior is good or poor, and, therefore, it is also difficult to give a driver feedback on what can be improved, at the same time as it is difficult for a vehicle owner to assess whether the vehicle is driven in a satisfactory, i.e. economical, fashion.

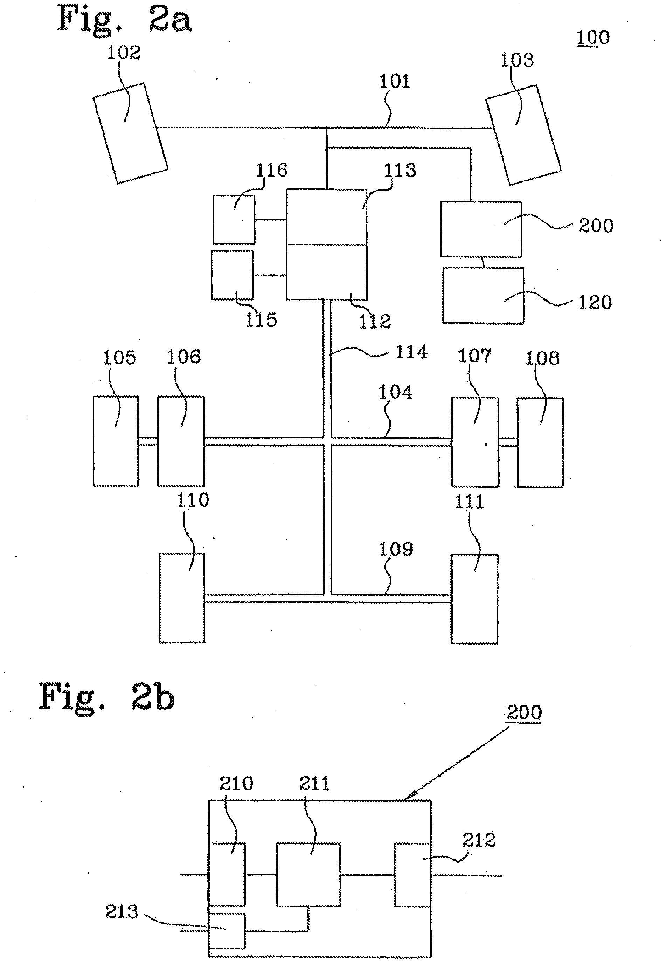

The present invention provides a device for evaluating the vehicle driver's anticipation ability, where a driver's ability to handle a vehicle in every given traffic situation can be assessed by evaluating vehicle internal data such as accelerator pedal position, brake pedal position and surroundings information from, for example, the type of radar that is used by adaptive cruise controls. The invention is explained more in detail with reference to fig. 2a-b, where in fig. 2a the vehicle in fig. 1 is shown being provided with a device 200 according to the invention.

The device 200 is shown more in detail in fig. 2b and comprises means 210 for receiving signals representing accelerator pedal position and brake pedal position, and if required convert these signals to a format adapted for a data processing unit 211. These signals can consist of a representation of the actual position of the pedals, which can be read using an appropriate sensor, or a calculated "position" based on a, by means of a measure taken by the driver, added brake torque or requested motor torque. For example, the position of a brake control, set by the driver, for a certain brake system can, by means of the construction of the vehicle, be arranged to be translated into an electronic signal, which is then treated by the control system of the vehicle to, based on this signal, apply a corresponding brake torque, i.e. today's heavy vehicles are often constructed in a such manner that the brake torque requested by the driver i.e. via a brake pedal, is translated into an electrical representation of how large part of the brake system's total brake capacity that is to be applied and which is then communicated to the above-mentioned brake management unit 120, usually via a data bus by means of which data can be communicated between the control units of the vehicle. Alternatively, the brake management unit 120 can perform said signal transformation and, if required, make these signals accessible on a data bus. Data can, for example, be transmitted on any of the data bus formats CAN (Controller Area Network), TTCAN or FlexRay, where the signals can be transmitted on a common data transmission format. Correspondingly, a representation of accelerator pedal/ accelerator control position can be made accessible on the data bus, for example for being provided to the above-mentioned engine management unit 116. Consequently, existing vehicles already today often comprise functionality to make available information regarding brake pedal/control position and accelerator pedal/control position on a vehicle data bus. The means 210 of the device 200 can thereby constitute a data bus connection for reception of signals representing control positions. The received signals are then added to a data processing unit 211, the function of which being described below.

The data processing unit 211 can, for example, consist of a processor, which is controlled by means of operation instructions, such as a computer program generated by means of an appropriate programming language, and being stored in a storage means, said storage means being built-in or connected to the processor. Said storage means can, for example, consist of one or more from the group: ROM (Read-Only Memory), PROM (Programmable Read-Only Memory), EPROM (Erasable PROM), Flash-memory, EEPROM (Electrically Erasable PROM). The device 200 can consist of a data processing unit with both integrated memory and integrated bus interface for a data bus of the above type and therefore be constructed in very compact manner. The device 200 further comprises output means 212 for output of processed data, for example for continuously transmitting data to a fleet management central or for presentation to the vehicle driver. Instead of the device receiving pedal positions via a data bus according to the above, said means 210 can, instead, consist of connection points for reception of sensor signals representing pedal positions. The device can, therefore, comprise means such as A/D-converters or receivers for receiving wirelessly transmitted sensor signals to convert received signals to a common format, which is adapted to said data processing unit 211.

A vehicle of the kind shown in fig. 1 normally comprises a plurality of brake systems, such as retarder, exhaust brake, service (foot) brake and motor brake and said means 210 can, consequently, comprise means for receiving signals representing control setting, and thereby the requested brake torque for each brake system, respectively, with regard to various kinds of driver actuatable brake systems.

Further, the device 200 includes means 213 for receiving traffic situation parameters according to the below in order to provide these to the data processing unit 211 for use according to the following.

The term traffic situation is, in the present invention, intended to mean traffic environment, or a combination of traffic environment and one or more vehicle specific parameters such as, but not limited to, own vehicle speed, acceleration and vehicle weight as well as the relative distance, speed and acceleration of surrounding vehicles (measured with radar). A traffic environment can be described in terms of, for example, crossing, roundabout, straight stretch, curve, uphill slope, downhill slope, top of a hill, valley, undulated or level highway or motorway, which will be described more closely below.

This information also is usually already accessible by means of data bus networks according to the above, and can, accordingly, e.g. be provided to the data processing unit 211 via said receiving means, or via separate means for each sensor or sensor type, for conversion to a format suitable for the data processing unit.

The data processing unit 211 comprises means for registering the time and/or distance the vehicle has traveled between a point in time when a measure taken by the driver to obtain a motor torque ceases until the point in time at which the driver takes a measure to apply a braking torque (or the other way around).

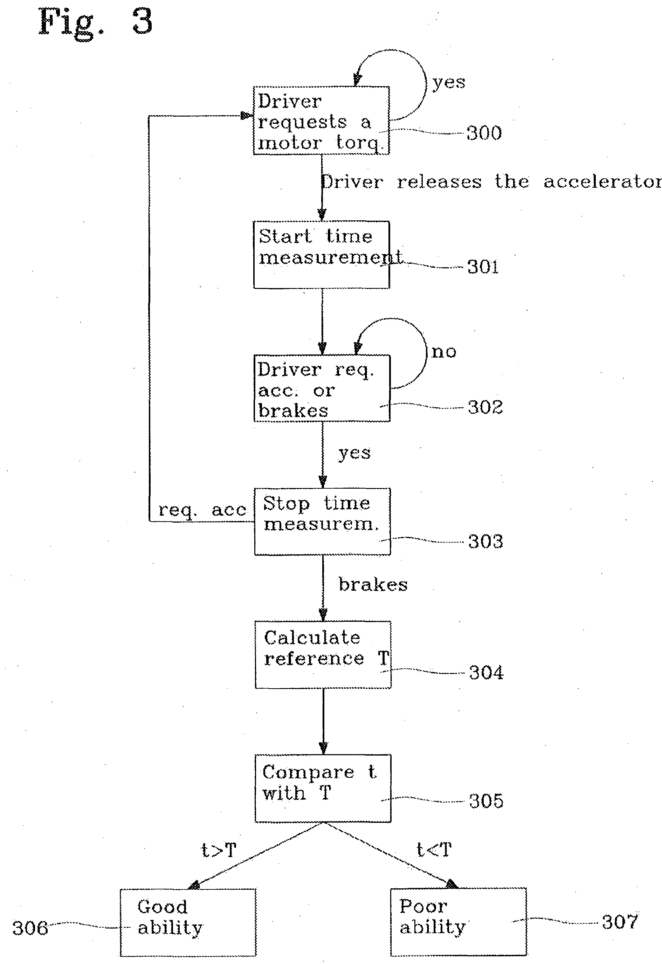

In fig. 3 is shown a flow chart exemplifying an embodiment of the invention. The process begins in step 300, wherein the process remains for as long as the driver requests a (positive) motor torque by pressing an accelerator pedal or maneuvering another type of accelerator control. As was mentioned, a detection of whether the driver is requesting a motor torque can be read via the control system of the vehicle or by reading an actual pedal/control position. If the data processing unit 211, in step 300, detects that the driver releases the accelerator pedal, i.e. the request for motor torque ceases, the process continues to step 301, where a time measurement is started or, alternatively, or in addition thereto, a distance measurement can be started, whereby the process continues to step 302 where it is determined whether the driver again requests a positive motor torque by pressing an accelerator pedal (or maneuvering of another accelerator control), or if the driver requests a braking torque by activating any of the vehicle's brake systems, such as depressing a brake pedal or maneuvering, e.g., a retarder control. For as long as the driver neither requests a motor torque nor a braking torque, the process remains in step 302 with activated time/distance measurement. However, if it is detected that the driver requests an acceleration or brakes, the process continues to step 303 where time measurement (distance measurement) is stopped and the obtained time t (and/or distance s) is stored. If the process continued to step 303 because the driver requested an acceleration, the measurement value t (s) is set to zero and the process returns to step 300. If, however, the driver has requested a braking torque, the process continues to step 304, where it is calculated, or in other ways obtained, a reference value T representing a desired minimum time between request for acceleration and braking, which reference value T is determined based on the current traffic situation. The measured value is then, in step 305, compared to the reference value. If t > T, the behavior in step 306 is considered to show a good anticipation ability. If, however, t < T, the behavior is, in step 307, considered to show a poor anticipation ability. Accordingly, the data processing unit 211 can calculate an opinion about the driver's anticipation ability in the current traffic situation in real time. The result can be output by means of output means 212 to, e.g., a data bus to be provided to a display unit to be shown directly to the driver, for example in the form of improvement suggestions. Alternatively, the information for each torque change can be saved for future examination by, for example, the vehicle owner. The obtained determination can in this case be stored either alone, whereby the complete vehicle journey afterwards can be evaluated, and statistics be obtained about how often the driver has exhibited a good anticipation ability. Alternatively, the determination and/or the time (and/or the distance and preferably also the vehicle speed) between the torque changes (the transition from requested motor torque to requested braking torque) is stored together with a representation/data representing the specific circumstances that was present at the current traffic environment for the specific torque change.

In this manner, a very accurate evaluation of the driver during a complete or a part of a journey can afterwards be performed, where, for example, it can be evaluated at which type of situations the driver exhibits an especially good/poor anticipation ability. For example, the driver's behavior can be divided into each separate surroundings situation, such as right-hand turn in an urban environment or passage of a top of a hill on a highway.

Very short requested braking actions in direct connection to a torque take off does not have to be assessed as negative, since such a braking action can be considered to be intended to deactivate a torque requesting system, e.g., a constant-speed cruise control. By comparing a short braking action with vehicle internal information about, for example, the status of the cruise control going from "active" to "passive", the intention to deactivate a system can be secured.

The length of the reference time T can, according to the above, depend on the traffic situation. This can consist of traffic environment and speed of the vehicle. For example, the shortest desired reference time T can be obtained as:

$$ T = f(surroundings) \cdot g(speed) + k \text{ ,} $$

, where k is constant and f(surroundings) constitutes a function of the surroundings and represents the time slot demand of the surroundings. When driving on a freeway, a longer time slot is desired compared to an urban environment. g(speed) is a function of the speed; $g(v) = c_1 \cdot v^b$, where $c_1$ and b constitutes constants.

The method shown in fig. 3 is, above all, useful in registering trends in the driver's driving manner that indicates the extent of aggressiveness in the driving and the effects on fuel consumption. For example, it is favorable to release the accelerator pedal well before a roundabout or crossing to roll freely quite a distance before a braking torque is commanded. The manner in which this to be performed is, among other things, determined by the speed of possible vehicles in front, retardation and relative distance to the own vehicle.

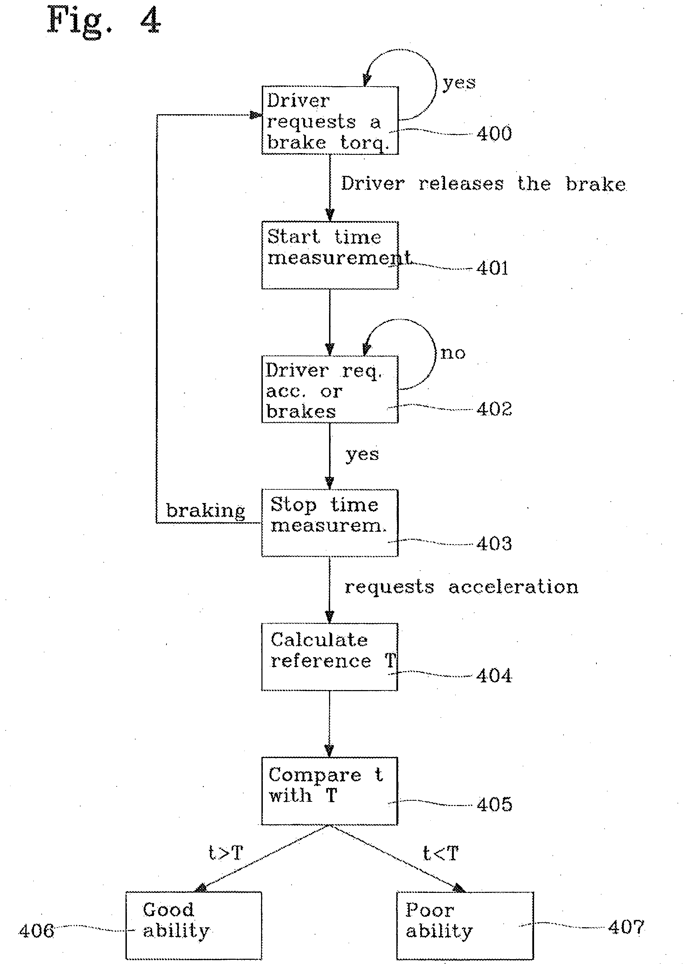

In fig. 4 is shown a flow chart that can be used at the opposite torque change, i.e. transition from request for a braking torque to request for a positive motor torque. In step 400 it is detected that the driver requests a braking torque, whereby it later in step 401 is started a time measurement (and/or distance measurement) when the driver stops the request for a braking torque, whereby the time measurement is stopped when it, in step 403, is established that the driver requests an acceleration or brakes. If the driver requests an acceleration, a reference value $T$ representing a desired minimum time between braking torque and positive motor torque based on the current traffic situation is calculated in step 404 according to the above. As before, the measured value is compared to the reference value (step 405), wherein if $t > T$ the behavior in step 406 is considered to exhibit a good anticipation ability. However, if $t < T$ the behavior is in step 407 considered to exhibit a poor anticipation ability.

Precisely as above, this can occur in real time and the result can either be shown directly for the driver or be saved for later examination.

Further, very short requested torque take offs in direct connection with a braking torque, should, in same manner as described above, not be considered as negative, since such a torque request can be considered to be intended to deactivate some retardation system, e.g. a constant-speed brake. In comparing a short motor torque take off with vehicle internal information, e.g. about the status of the constant speed brake changing from "active" to "passive", the deactivating intention can, in this case also, be secured.

The described method in fig. 4, results in a measurement of the driver's tendency towards aggressiveness in the driving, such as wearing brakes and using unnecessary amounts of fuel. For example, in a situation when catching up with an accelerating vehicle ahead, it is unfavorable to brake all the way to the vehicle ahead. Instead, the driver should brake somewhat earlier and let the vehicle roll for a longer distance before an accelerating torque is applied.

Naturally, the different above opinions can be combined and be subject to the forming of a mean value. For example, a plurality of such opinions can be subject to individual forming of a mean value in respective categories of traffic environment to obtain a measurement number that provides an overall picture of the driver's driving manner concerning aggressiveness, traffic security, fuel and vehicle economy.

With regard to the traffic environment, it is possible to obtain a very accurate representation of the surroundings of the vehicle. In the parallel European Patent Application with publication number EP2032941 entitled "Device and system for classifying vehicle surroundings" and having the same filing date as the present application, is described a solution for, during traveling, determining the type of a distance, which is divided into segments, of a route that a vehicle is traveling. According to EP2032941, it is provided a device for, during traveling with a vehicle, determining the type of a distance of a way along which the vehicle is traveling, wherein the distance is arranged to be divided into segments. The device comprises means for determining that a segment has been started, means for determining a reference direction of movement at the start of the segment, means for receiving at least one direction parameter value, which constitutes a representation of the direction of movement of the vehicle, or by which a representation of the direction of the movement of the vehicle can be determined, means for determining if the vehicle has reached the end of the segment, and means for determining the type of the segment from said received direction parameter values. This has the advantage that the journey of the vehicle can be divided in segments of different types.

The described solution can, in a very accurate manner, determine the type of a plurality of consecutive or adjacent segments, such as right-hand turn, left-hand turn, straight stretch, uphill slope, downhill slope, and, in particular together with time and/or speed data perform an accurate classification of the distance. The classification can, for example, consist of idle driving, power take off (PTO), marshalling, queue, city centre, urban area, undulated and/or straight or curved road, straight and level or undulated road.

The device 200 according to the present invention, can comprise means for receiving a surroundings classification from a device according to the said Patent Application. Alternatively, the device 200 according to the invention can comprise means for performing the described traffic surroundings classification. These means can, for example, consist of the data processing unit 211, whereby the device 200 is provided with means to be able to receive information that is necessary for the surroundings classification, for example via a data bus. In using a very accurate description of the traffic environment (vehicle surroundings) in this manner, the vehicle driver's anticipation ability can be assessed for each separate type of segment, such as left-hand turns or passage through roundabouts. At the said determination it can also be taken into consideration what the road looked like before, e.g. a roundabout, e.g., if it was straight, curved or undulated. Accordingly, this has as result that a very accurate analysis of the driver's behavior can be accomplished.

Other methods to assess the traffic environment comprise, for example, using positioning systems and map data and/or an inclinometer.

According to the above, the present invention can be used for real time feedback to the driver. The vehicle can, in this case, be provided with a display that constantly shows the driver's anticipation ability, for example, as a percentage between 0 and 100, where, for example, a result on 85 % and over indicates a very good anticipation ability. Accordingly, the driver can "compete" with himself/herself in driving safe and economical. The information can also be used by the driver's superiors to determine which driver that is in need for help to improve his/her driving, or to identify a driver who is doing particularly well and possibly reward him/her.

The present invention can further be combined with other systems for evaluating a vehicle driver.

In the parallel Swedish Patent Application 0601173-8 entitled "Anordning for bromsslitagebedömning (in English: Device for Brake Wear Assessment)" and having the same filing date as the present invention, it is described a solution to assess a vehicle driver's ability to use vehicle brakes. The present invention can advantageously be utilized together with the said solution.

Further, in the parallel Swedish Patent Application 0601174-6, entitled "Anordning for bestamning av bränsleförbrukningsbeteende (in English: Device for Determining an fuel consumption behaviour)" and having the same filing date as the present application, is described another system for driver evaluation, together with which the present invention advantageously can be utilized. This patent application discloses a device for evaluating a vehicle driver's ability to drive fuel efficient by studying the driver's consumption in special cases such as tops of hills, valleys and downhill slopes.

By combining a plurality of different procedures for evaluating a vehicle driver, a very accurate determination of the vehicle driver can, when put together, be performed.

As is realized, the vehicle can be arranged to continuously transmit data to a monitoring central, whereby the above evaluation can be performed in the monitoring central, instead of in the vehicle.

**Claims**

1. Device for determining a anticipation ability for a driver of a vehicle in a traffic situation, wherein the device comprises:

　- means for determining if the vehicle driver is taking a first measure, wherein said first measure is a vehicle braking measure, or a measure for output of positive motor torque,
　- means for determining a first time at which the said measure is finished,
　- means for determining a second time at which the driver takes a measure counteracting said finished measure, and
　- means for receiving at least one reference parameter value representing the vehicle surroundings or at least one parameter value from at least one vehicle internal sensor with which a reference parameter value that is representative for the vehicle surroundings can be determined,
　- means for determining a reference direction of motion,
　**characterized in**
　- means for comparing a value representing the difference between the said second time and the said first time with the reference parameter value, and based on the comparison determine a parameter value representing the driver's anticipation ability,
　- means for dividing a distance of the way a vehicle travels into segments,
　- means for determining that a segment is started,
　- means for, during said segment, receiving at least one direction parameter value, which consists of a representation of the vehicle's direction of motion, or with which a representation of the vehicle's direction of motion can be determined,
　- means for determining that the vehicle has reached the end of the segment,
　- means for, from the said received direction parameter values determining the type of the segment, and
　- means for determining the vehicle surroundings by means of one or a plurality of consecutive or adjacent segments.

2. Device according to claim 1, where said measure for take off of a positive motor torque is a throttle control actuating measure.

3. Device according to claim 1 or 2, where said difference consists of a representation of the time or distance that has been passed between the ceasing of taking the said first measure and the taking of the said second measure.

4. Device according to any of the claims 1-3, wherein said first and second measure, respectively, is taken by means of at least one from the group: accelerator pedal, accelerator control, brake pedal, brake control, cruise control, retarder, constant-speed brake.

5. Device according to claim 1, **characterized in** said parameter values are being generated by vehicle internal sensors.

6. Device according to claim 1 or 5, **characterized in** said segment type being any from the group: right-hand turn, left-hand turn, right-hand curve, left-hand curve, straight stretch, roundabout, top of a hill, valley, crossing, right-hand turn in crossing, left-hand turn in crossing, level road, uphill slope, downhill slope.

**7.** Device according to any of the preceding claims, wherein the device comprises means for storing said parameter value representing the driver's anticipation ability together with a representation of said vehicle surroundings.

**8.** Device according to claim 7, where the device comprises means for storing said data for a plurality of traffic situations.

**9.** Device according to any of the claims 1-8, **characterized in that** it comprises means for transmitting said parameter value representing the driver's anticipation ability, to display means arranged in said vehicle.

**10.** Device according to any of the claims 1-9, **characterized in that** it comprises means for transmitting said parameter value representing the driver's anticipation ability, to a remotely located monitoring central.

**11.** Device according to any of the preceding claims, **characterized in that** one or a plurality of said means at least partially consist of a computer program, wherein the device at least partially consists of a computer program product comprising a computer-readable medium and a computer program, wherein the computer program is being comprised in the computer-readable medium.

**12.** Device according to claim 11, **characterized in that** said computer-readable medium consists of one or more of the group: ROM (Read-Only Memory), PROM (Programmable Read-Only Memory), EPROM (Erasable PROM), Flash memory, EEPROM (Electrically Erasable PROM).

**13.** Vehicle, **characterized in that** it comprises a device according to any of the claims 1-12.

**Patentansprüche**

**1.** Vorrichtung zum Ermitteln einer Voraussehensfähigkeit für einen Fahrer eines Fahrzeugs in einer Verkehrssituation, wobei die Vorrichtung umfasst:

     - Mittel zum Ermitteln, ob der Fahrzeugführer eine erste Maßnahme vornimmt, wobei die erste Maßnahme eine Fahrzeugbremsmaßnahme oder eine Maßnahme zum Ausgeben von positivem Motordrehmoment ist,
     - Mittel zum Ermitteln eines ersten Zeitpunkts, an dem die Maßnahme abgeschlossen ist,
     - Mittel zum Ermitteln eines zweiten Zeitpunkts, an dem der Fahrer eine der abgeschlossenen Maßnahme entgegenwirkende Maßnahme vornimmt, und
     - Mittel zum Empfangen mindestens eines die Fahrzeugumgebung darstellenden Referenzparameterwerts oder mindestens eines Parameterwerts von mindestens einem fahrzeuginternen Sensor, mit dem ein Referenzparameterwert ermittelt werden kann, der darstellend für die Fahrzeugumgebung ist,
     - Mittel zum Ermitteln einer Referenzbewegungsrichtung,
     **gekennzeichnet durch**
     - Mittel zum Vergleichen eines die Differenz zwischen dem zweiten Zeitpunkt und dem ersten Zeitpunkt darstellenden Werts mit dem Referenzparameterwert, und, basierend auf dem Vergleich, Ermitteln eines die Voraussehensfähigkeit des Fahrers darstellenden Parameterwerts,
     - Mittel zum Aufteilen einer Strecke des von einem Fahrzeug befahrenen Weges in Abschnitte,
     - Mittel zum Ermitteln, dass ein Abschnitt beginnt,
     - Mittel zum Empfangen mindestens eines Richtungsparameterwerts während des Abschnitts, der aus einer Darstellung der Bewegungsrichtung des Fahrzeugs besteht oder mit dem eine Darstellung der Bewegungsrichtung des Fahrzeugs ermittelt werden kann,
     - Mittel zum Ermitteln, dass das Fahrzeug das Ende des Abschnitts erreicht hat,
     - Mittel zum Ermitteln der Art des Abschnitts aus den empfangenen Richtungsparameterwerten, und
     - Mittel zum Ermitteln der Fahrzeugumgebung anhand von einem oder mehreren aufeinander folgenden oder benachbarten Abschnitten.

**2.** Vorrichtung nach Anspruch 1, wobei die Maßnahme zum Ausgeben eines positiven Motordrehmomentes eine Drosselsteuerungsbetätigungsmaßnahme ist.

**3.** Vorrichtung nach Anspruch 1 oder 2, wobei die Differenz aus einer Darstellung der Zeit oder der Entfernung, die zwischen dem Beenden des Vornehmens der ersten Maßnahme und dem Vornehmen der zweiten Maßnahme befahren wurde, besteht.

**4.** Vorrichtung nach einem der Ansprüche 1 bis 3, wobei die erste und zweite Maßnahme entsprechend mittels mindestens eines aus der folgenden Gruppe vorgenommen wird: ein Gaspedal, eine Beschleunigersteuerung, ein Bremspedal, eine Bremssteuerung, ein Tempomat, ein Retarder, eine Konstantgeschwindigkeitsbremse.

**5.** Vorrichtung nach Anspruch 1, **dadurch gekennzeichnet, dass** die Parameterwerte von fahrzeuginternen Sensoren erzeugt werden.

**6.** Vorrichtung nach Anspruch 1 oder 5, **dadurch gekennzeichnet, dass** die Abschnittsart eine aus der folgenden Gruppe ist:

ein Rechtsabbiegen, ein Linksabbiegen, eine Rechtskurve, eine Linkskurve, eine gerade Strecke, ein Kreisverkehr, eine Spitze eines Hügels, ein Tal, eine Kreuzung, ein Rechtsabbiegen in einer Kreuzung, ein Linksabbiegen in einer Kreuzung, eine ebene Straße, ein Anstieg oder ein Gefälle.

**7.** Vorrichtung nach einem der vorhergehenden Ansprüche, wobei die Vorrichtung Mittel zum Speichern des die Voraussehensfähigkeit des Fahrers darstellenden Werts zusammen mit einer Darstellung der Fahrzeugumgebung umfasst.

**8.** Vorrichtung nach Anspruch 7, wobei die Vorrichtung Mittel zum Speichern der Daten für mehrere Verkehrssituationen umfasst.

**9.** Vorrichtung nach einem der Ansprüche 1 bis 8, **dadurch gekennzeichnet, dass** sie Mittel zum Übertragen des die Voraussehensfähigkeit des Fahrers darstellenden Parameterwerts an Anzeigemittel umfasst, die in dem Fahrzeug angeordnet sind.

**10.** Vorrichtung nach einem der Ansprüche 1 bis 9, **dadurch gekennzeichnet, dass** sie Mittel zum Übertragen des die Voraussehensfähigkeit des Fahrers darstellenden Parameterwerts an eine entfernt befindliche Überwachungszentrale umfasst.

**11.** Vorrichtung nach einem der vorhergehenden Ansprüche, **dadurch gekennzeichnet, dass** eines oder mehrere der Mittel mindestens teilweise aus einem Computerprogramm bestehen, wobei die Vorrichtung mindestens teilweise aus einem ein computerlesbares Medium und ein Computerprogramm umfassenden Computerprogrammprodukt besteht, wobei das Computerprogramm in dem computerlesbaren Medium umfasst ist.

**12.** Vorrichtung nach Anspruch 11, **dadurch gekennzeichnet, dass** das computerlesbare Medium aus einem oder mehreren aus der folgenden Gruppe besteht: Festwertspeicher (Read-Only Memory, ROM), programmierbarer Festwertspeicher (Programmable Read-Only Memory, PROM), löschbarer Festwertspeicher (Erasable PROM, EROM), Flash-Speicher, elektrisch löschbarer Festwertspeicher (Electrically Erasable PROM, EEPROM).

**13.** Fahrzeug, **dadurch gekennzeichnet, dass** es eine Vorrichtung nach einem der Ansprüche 1 bis 12 umfasst.

## Revendications

**1.** Dispositif pour déterminer la capacité d'anticipation d'un conducteur de véhicule en situation de circulation, le dispositif comportant :

- des moyens pour déterminer si le conducteur du véhicule est en train de prendre une première mesure, ladite première mesure étant une mesure de freinage du véhicule, ou une mesure pour générer en sortie un couple moteur positif,
- des moyens pour déterminer un premier temps auquel ladite mesure est finie,
- des moyens pour déterminer un deuxième temps auquel le conducteur prend une mesure contrecarrant ladite mesure finie, et
- des moyens pour recevoir au moins une valeur de paramètre de référence représentant l'environnement du véhicule ou au moins une valeur de paramètre provenant d'au moins un capteur interne au véhicule, grâce auquel une valeur de paramètre de référence qui est représentative de l'environnement du véhicule peut être déterminée,
- des moyens pour déterminer une direction de déplacement de référence,
**caractérisé par**
- des moyens pour comparer une valeur représentant la différence entre ledit deuxième temps et ledit premier temps à la valeur du paramètre de référence, et, en fonction de la comparaison, déterminer une valeur du paramètre représentant la capacité d'anticipation du conducteur,
- des moyens pour diviser une distance du trajet que parcourt un véhicule en segments,
- des moyens pour déterminer qu'un segment est commencé,
- des moyens, actifs sur ledit segment, pour recevoir au moins une valeur de paramètre de direction, constituée d'une représentation de la direction de déplacement du véhicule, ou grâce auxquels une représentation de la direction de déplacement du véhicule peut être déterminée,
- des moyens pour déterminer que le véhicule a atteint la fin du segment,
- des moyens pour déterminer, à partir desdites valeurs de paramètre de direction reçues, le type du segment, et
- des moyens pour déterminer l'environnement du véhicule à l'aide d'un ou d'une pluralité de segments consécutifs ou adjacents.

**2.** Dispositif selon la revendication 1, dans lequel ladite mesure pour générer un couple moteur positif est une mesure d'actionnement de la commande d'accélérateur.

**3.** Dispositif selon la revendication 1 ou 2, dans lequel ladite différence est constituée par une représentation du temps ou de la distance qui se sont écoulés entre l'arrêt de l'exécution de ladite première mesure et l'exécution de ladite deuxième mesure.

**4.** Dispositif selon l'une quelconque des revendications 1 à 3, dans lequel lesdites première et deuxième mesures, respectivement, sont exécutées à l'aide d'au moins l'un du groupe : pédale d'accélérateur, commande d'accélérateur, pédale de frein, commande de frein, commande du régulateur de vitesse, frein moteur, frein vitesse constante.

**5.** Dispositif selon la revendication 1, **caractérisé en ce que** lesdites valeurs de paramètre sont générées par des capteurs internes au véhicule.

**6.** Dispositif selon la revendication 1 ou 5, **caractérisé en ce que** ledit type de segment est l'un quelconque du groupe : virage à droite, virage à gauche, courbe à droite, courbe à gauche, portion de ligne droite, rond-point, sommet, creux, croisement, virage à droite dans un croisement, virage à gauche dans un croisement, route plate, pente montante, pente descendante.

**7.** Dispositif selon l'une quelconque des revendications précédentes, dans lequel le dispositif comprend des moyens pour mémoriser ladite valeur de paramètre représentant la capacité d'anticipation du conducteur avec une représentation dudit environnement du véhicule.

**8.** Dispositif selon la revendication 7, dans lequel le dispositif comprend des moyens pour mémoriser lesdites données pour une pluralité de situations de circulation.

**9.** Dispositif selon l'une quelconque des revendications 1 à 8, **caractérisé en ce qu'**il comprend des moyens pour transmettre ladite valeur de paramètre représentant la capacité d'anticipation du conducteur à des moyens d'affichage disposés dans ledit véhicule.

**10.** Dispositif selon l'une quelconque des revendications 1 à 9, **caractérisé en ce qu'**il comprend des moyens pour transmettre ladite valeur de paramètre représentant la capacité d'anticipation du conducteur à un central de contrôle situé à distance.

**11.** Dispositif selon l'une quelconque des revendications précédentes, **caractérisé en ce que** l'un ou une pluralité desdits moyens est au moins partiellement constitué d'un programme informatique, le dispositif étant au moins partiellement constitué d'un produit informatique comprenant un support lisible par un ordinateur et un programme informatique, le programme informatique étant inclus dans le support lisible par un ordinateur.

**12.** Dispositif selon la revendication 11, **caractérisé en ce que** ledit support lisible par un ordinateur est constitué d'un ou plusieurs élément(s) du groupe : ROM (mémoire morte), PROM (mémoire morte programmable), EPROM (mémoire morte programmable effaçable), mémoire flash, EEPROM (mémoire morte programmable effaçable électriquement).

**13.** Véhicule, **caractérisé en ce qu'**il comprend un dispositif selon l'une quelconque des revendications 1 à 12.

# Fig. 1

# Fig. 2a

# Fig. 2b

# Fig. 3

```
┌─────────────┐  yes
│ Driver      │◄─┐
│ requests a  │──┘ ──300
│ motor torq. │
└─────────────┘
       │  Driver releases the accelerator
       ▼
┌─────────────┐
│ Start time  │
│ measurement │ ──301
└─────────────┘
       │
       ▼
┌─────────────┐  no
│ Driver req. │◄─┐
│ acc. or     │──┘ ──302
│ brakes      │
└─────────────┘
       │  yes
       ▼
┌─────────────┐
│ Stop time   │ ──303
│ measurem.   │
└─────────────┘
  req. acc
       │  brakes
       ▼
┌─────────────┐
│ Calculate   │
│ reference T │ ──304
└─────────────┘
       │
       ▼
┌─────────────┐
│ Compare t   │
│ with T      │ ──305
└─────────────┘
   t>T    t<T
   ╱        ╲
┌─────────┐  ┌─────────┐
│ Good    │  │ Poor    │
│ ability │  │ ability │ ──307
└─────────┘  └─────────┘
306
```

# Fig. 4

```
        ┌──────────┐ ──┐ yes
        │ Driver   │   │
   ┌───▶│ requests a│◀──┘
   │    │ brake torq.│──── 400
   │    └──────────┘
   │         │ Driver releases the brake
   │         ▼
   │    ┌──────────┐
   │    │ Start time│
   │    │ measurement│──── 401
   │    └──────────┘
   │         │
   │         ▼
   │    ┌──────────┐ ──┐ no
   │    │ Driver req.│  │
   │    │ acc. or  │◀──┘
   │    │ brakes   │──── 402
   │    └──────────┘
   │         │ yes
   │         ▼
   │ braking ┌──────────┐
   └─────────│ Stop time│
             │ measurem.│──── 403
             └──────────┘
                  │ requests acceleration
                  ▼
             ┌──────────┐
             │ Calculate│
             │ reference T│──── 404
             └──────────┘
                  │
                  ▼
             ┌──────────┐
             │ Compare t│
             │ with T   │──── 405
             └──────────┘
            t>T  ╱    ╲  t<T
               ▼        ▼
      ┌──────────┐  ┌──────────┐
406 ──│ Good     │  │ Poor     │──── 407
      │ ability  │  │ ability  │
      └──────────┘  └──────────┘
```

**EP 2 033 167 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0007150 A
- DE 10056756
- WO 0186459 A1
- EP 2032941 A
- SE 06011738
- SE 06011746